(19) Europäisches Patentamt

European Patent Office

Office européen des brevets

(11) Publication number: **0 285 143 B1**

(12) **EUROPEAN PATENT SPECIFICATION**

(45) Date of publication of patent specification: 30.06.93

(51) Int. Cl.⁵: **C12P 21/08**, G01N 33/574, C12N 5/18, A61K 39/395, C12P 21/00, C12R 1/91

(21) Application number: 88105199.9

(22) Date of filing: 30.03.88

(54) Antihuman pulmonary adenocarcinoma monoclonal antibody.

(30) Priority: 31.03.87 JP 78186/87

(43) Date of publication of application:
05.10.88 Bulletin 88/40

(45) Publication of the grant of the patent:
30.06.93 Bulletin 93/26

(84) Designated Contracting States:
DE FR GB

(56) References cited:
EP-A- 0 218 257
EP-A- 0 252 769
GB-A- 2 186 885

CANCER RESEARCH, vol. 44, no. 2, February 1984, pages 681-687, Philadelphia, US; N.M. VARKI et al.: "Antigens associated with a human lung adenocarcinoma defined by monoclonal antibodies"

BEHRING INSTITUTE MITTEILUNG, no. 74, 1984, pages 27-34, Marburg, DE; K. BOSSLET et al.: "Molecular characteristics of two lung carcinoma cell-line associated membrane antigens"

(73) Proprietor: KYOWA HAKKO KOGYO CO., LTD.
Ohtemachi Bldg., 6-1 Ohtemachi I-chome
Chiyoda-ku Tokyo 100(JP)

(72) Inventor: Yoshida, Hajime
9-18, Isobe
Sagamihara-shi Kanagawa(JP)
Inventor: Shitara, Kenya
4-17-9, Morino
Machida-shi Tokyo(JP)

(74) Representative: Kinzebach, Werner, Dr. et al
Patentanwälte Reitstötter, Kinzebach und
Partner Sternwartstrasse 4 Postfach 86 06 49
W-8000 München 86 (DE)

**Description**

FIELD OF THE INVENTION

This invention relates to a monclonal antibody ALC-390, which belongs to the IgG class and is reactive to human pulmonary adenocarimona and recognizes a protein epitope as the antigenic determinant, as well as to processes for detecting and treating pulmonary adenocarcinoma by applying the antibody. The present invention is applicable to the pathological diagnosis and treatment of pulmonary adonocarcinoma and thus useful in the field of diagnostic and therapeutic agents.

BACKGROUND OF THE INVENTION

N. M. Varki et al. disclose in Cancer Research (1984), 44, 681-687, monoclonal antibodies KS 1/4, KS 1/9 and KS 1/17, recognizing antigens associated with human lung adenocarcinoma. Monoclonal antibodies KS 1/4 and KS 1/17 recognize similar glycoprotein antigens on the lung carcinoma cells while antibody KS 1/9 appears to detect a glycolypid antigen. As stated in table 2 of this reference antibodies KS 1/4 and KS 1/17 show a strong reaction with lung small-cell carcinoma cells.

K. Bosselt et al. disclose in Behring Inst. Mitt. (1984) 74, 27-34, monoclonal antibodies BW 181/26 and BW 166/6 directed to lung carcinoma cell line associated membrane antigens. As disclosed in table 1a of this reference, antibody BW 181/26 reacts with different lung tumor cell lines. As further disclosed on page 29 of this reference, the monoclonal antibody BW 166/6 is induced against the "Chago" large cell lung carcinoma cell line.

Although it seems that there is no excellent tumor marker for lung cancer, we have developed a process for efficiently producing an antihuman pulmonary adenocarimona monoclonal antibody by using human normal lung tolerant mice (cf. Japanese Patent Application (OPI) No. 190721/85) (the term "OPI" used herein means an unexamined published application.) and thus established antihuman pulmonary adenocarcinoma monoclonal antibodies useful for serodiagnosis of pulmonary adenocarcinoma including SLC-1 (cf. Japanese Patent Application (OPI) No. 234357/86), ALC-1 (cf. Japanese Patent Application (OPI) No. 234358/86), ALC-186 (cf. Japanese Patent Application (OPI) No. 80558/87) and ALC-454 (cf. Japanese Patent Application (OPI) No. 19561/88 corresponding to EP-A-0252769). (The term "OPI" used herein means an unexamined publication.). However, each of these monoclonal antibodies, which would recognize an antigen released in the serum of a patient suffering from lung cancer, is not always suitable for the cytological or histlogical diagnosis or treatment of this disease, though it is useful in the serodiagnosis thereof. Namely, a monoclonal antibody which recognized not a released antigen but rather a cancer cell membrane surface antigen is preferable for these purposes.

SUMMARY OF THE INVENTION

As described above, no monoclonal antibody to date is known which is highly specific to a pulmonary adenocarcinoma cell membrane and reacts with it at a high ratio. Thus, it is highly advantageous from diagnostic and therapeutic viewpoints to establish a monoclonal antibody highly specific to lung cancer cell membranes.

We have found that the monoclonal antibody ALC-390, which is produced by a hybridoma established by using a human pulmonary adenocarcinoma membrane component as an immunogen, is highly specific to a pulmonary adenocarcinoma cell membrane and is thus remarkably useful in the pathological diagnosis and treatment of pulmonary adenocarcinoma.

BRIEF DESCRIPTION OF THE DRAWING

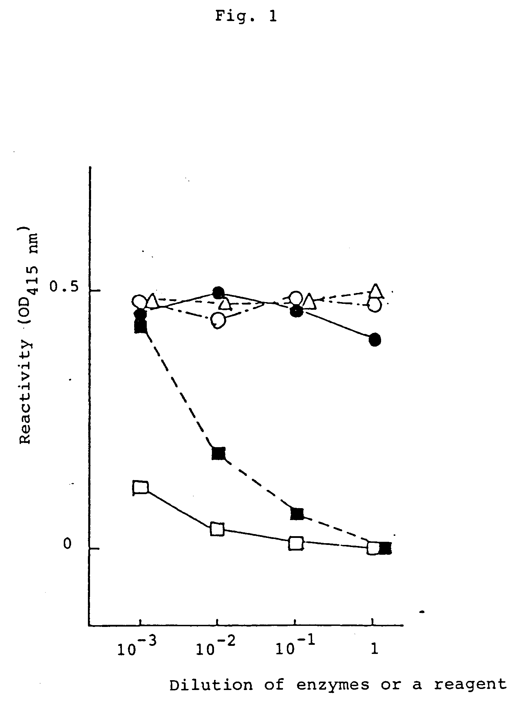

Fig. 1 shows a result of analysis of the antigen determinant recognized by the monoclonal antibody ALC-390.

In Fig. 1, O—O shows a result of treatment with neuraminidase (0.1 U/mℓ); △—△ shows a result of treatment with $\alpha$-L-fucosidase (0.1 U/mℓ); ■—■ shows a result of treatment with trypsin (0.25 w/v%); □—□ shows a result of treatment with protease (10 U/mℓ); and ●—● shows a result of treatment with NaIO₄ (50 mM).

DETAILED DESCRIPTION OF THE INVENTION

The present invention provides an antihuman pulmonary adenocarcinoma monoclonal antibody which is obtained by fusing mouse spleen cells immunized with a human pulmonary adenocarcinoma tissue membrane component with mouse myeloma cells to give hybridomas; selecting a monoclonal antibody reactive to a human pulmonary adenocarcinoma cell membrane; incubating the selected hybridomas in a medium or administering the same to a mouse; and then collecting the desired monoclonal antibody from the incubation medium or from the ascites fluid of the mouse.

The monoclonal antibody ALC-390 (ECACC 87032601) of the present invention belongs to the IgG class, reacts with pulmonary adenocarcinoma cell membrane and recognizes a protein epitope thereof as an antigenic determinant.

Another aspect of the invention relates to a method of detecting the presence of human pulmonary adenocarcinoma in a patient sample comprising:

(a) subjecting a serum sample from the patient to an enzyme immunoassay with the monoclonal antibody ALC-390 (ECACC 87032601) that (1) belongs to the class IgG, (2) recognizes a protein epitope as the antigenic determinant and (3) is reactive with human pulmonary adeoncarcinoma cell membranes and (4) is devoid of reactivity with normal human lung tissue; and thereafter

(b) examining the results for the presence of a positive reaction.

Yet another aspect is a diagnostic method of distinguishing human pulmonary adonocarcinoma cells from other cells including lung squamous cell carcinoma cells lung large cell carcinoma cells, lung small cell carcinoma cells and normal human lung cells, said diagnostic method comprising the steps of:

(a) subjecting a patient sample to an enzyme immunoassay with the monoclonal antibody ALC-390 (ECACC 87032601) that (1) belongs to the class IgG, (2) selectively recognizes a protein epitope as the antigenic determinant and (3) is substantially more reactive with human pulmonary adenocarcinoma cell membranes than lung squamous cell carcinoma cells, lung large cell carcinoma cells, lung small cell carcinoma cells and thereafter

(b) assessing the degree of reactivity, if any, of the monoclonal antibody with the cells of the patient sample.

The present invention also provides a pharmaceutical composition comprising an effective amount of the above-mentioned antibody optionally in combination with a pharmaceutically acceptable carrier or adjuvant.

The present invention also relates to the use of the claimed antibody for preparing a pharmaceutical composition for the treatment of pulmonary adenocarcinoma.

Examples of the monoclonal antibody of the present invention include ALC-390 which is produced by a hybridoma cell strain ALC-390 deposited with European Collection of Animal Cell Culture in England on March 26, 1987 as ECACC No. 87032601 under the Budapest treaty.

A process for producing the monoclonal antibody of the present invention will now be described in detail.

(1) Immunization of animal and preparation of antibody-producing cells.

Mice aged three to ten weeks, preferably eight weeks, are immunized with human pulmonary adenocarcinoma cells, or a tissue or membrane component thereof, to prepare antibody-producing cells in the spleen, lymph nodes and peripheral blood of each mouse. It is preferable that the mice to be immunized are preliminarily treated with human normal lung cells to make the aminals immunologically tolerant. The immunization may be effected by subcutaneously, intravenously or intraperitoneally administering $10^6$ to $10^7$ human pulmonary adenocarcinoma cells, a human pulmonary adenocarcinoma tissue or membrane components thereof (10 to 500 $\mu$g) to each animal together with an appropriate adjuvant such as complete Freund's adjuvant or aluminum hydroxide gel combined with B. pertussis vaccine. The antigen is again administered to the animal two to five times at one to two week intervals. Three to seven days after each immunization, the blood of the animal is collected from the fundus plexus venosus and the reaction of the collected serum with human pulmonary adenocarcinoma is examined by, for example, the following enzyme immunoassay method (cf. Enzyme-linked Immunosorbent Assay (ELISA), published by Igaku Shoin, 1976).

Enzyme immunoassay:

100 to 200 $\mu\ell$ portions of membrane components of normal or tumor cells, i.e., membrane fragments, each containing 10 to 1,000 $\mu$g/m$\ell$ of proteins are pipetted into a 96-well EIA plate manufactured by Flow Laboratories. After allowing the plate to stand at 4 °C for one to two days, the supernatant in each well is removed and the residue is thoroughly washed with deionized water or a phosphate buffer solution (PBS: comprising 1.83 g of disodium phosphate, 0.21 g of monopotassium phosphate, 7.65 g of sodium chloride and 1 $\ell$ of distilled water, pH 7.2). Then, 100 to 200 $\mu\ell$ of a 1 w/v% solution of bovine serum albumin (BSA) in PBS is pipetted into each well and the plate is allowed to stand at 4 °C for one to two days to thereby block the binding residues with proteins remaining on the plate. Then, the BSA/PBS is discarded and the residue is thoroughly washed with deionized water or PBS. Subsequently, 100 $\mu\ell$ portions of samples, each used as the primary antibody and obtained by diluting mouse serum, the hybridoma incubation supernatant or crude monoclonal antibody with BSA/PBS, are pipetted into each well and the plate is allowed to stand at 4°C overnight. After washing the plate with deionized water once followed by washing with a 2 M NaCl solution six times, 100 $\mu\ell$ of rabbit antimouse immunoglobulin/peroxidase complex (manufactured by DAKO and marketed by Kyowa-Medex) diluted 100-fold are pipetted into each well and then the plate is allowed to stand at room temperature for two hours.

After thoroughly washing the plate with PBS, the coloration is determined by the absorbance at $OD_{415nm}$ with the use of an ABTS substrate solution which is obtained by dissolving 550 mg of diammonium 2,2'-azinobis(3-ethylbenzothiazoline-6-sulfonate in 1 $\ell$ of a 0.1 M citrate buffer solution (pH 4.2) and adding 1 $\mu\ell$/m$\ell$ of hydrogen peroxide thereto immediately before use. Then, a mouse showing an intense reactivity with pulmonary adenocarcinoma cells or tissue or membrane component thereof is employed as a human pulmonary adenocarcinoma immunized mouse providing antibody-producing cells in the preparation of the hybridoma.

When cells per se are to be used as an antigen, the enzyme immunoassay is carried out by incubating target cells on an Falcon 3072 plate; adding 0.25 v/v% glutaraldehyde in PBS thereto; allowing the plate to stand at room temperature for one to two hours; thoroughly washing the same with PBS; further adding 100 to 200 $\mu\ell$ of 1 w/v% BSA in PBS; thoroughly washing the same with deionized water or PBS; and determining the antibody titer in the same manner as the use of the coat plate as obtained above.

Three to four days before cell fusion, 2 to 5 × $10^6$ of human pulmonary adenocarcinoma cells or 20 to 400 $\mu$g of human pulmonary adenocarcinoma tissue or membrane components thereof are intraperitoneally administered to an immunized mouse. Then, the spleen of the animal is removed and minced in MEM (manufactured by Nissui Pharmaceutical Co., Ltd.). The pieces are loosened with a pincette and cetrifuged at 1,200 rpm for five minutes. After discarding the supernatant, the residue is treated with a tris/ammonium chloride buffer solution (pH 7.65) for one to two minutes to thereby remove erythrocytes. After washing with MEM three times, cells for fusion can be obtained.

The membrane component of a human pulmonary adenocarcinoma tissue is prepared in the following manner.

A tissue pieces stored at -80°C are thawed and cut into pieces with scissors. Then, the pieces are mechanically ground with an Ultra-Disperser (manufactured by Yamato), homogenized with a glass Teflon homogenizer and then cetrifuged at 100,000 × g for 20 minutes. The supernatant is further centrifuged at 4°C and at 100,000 × g for one hour and the precipitate thus obtained is suspended in a 0.01 M phosphate buffer solution (pH 7.2). This suspension is employed as the membrane component.

(2) Preparation of myeloma cells

An established cell line obtained from a mouse is employed as the source of myeloma cells. Examples of suitable myeloma cells include 8-azaguanine resistant mouse, originating from BALB/c, myeloma cell lines P3-X63Ag8-U1 (P3-U1) [cf. Current Topics in Microbiol. Immunol. 1; and Europ. J. Immunol., 6, 511-519 (1976)]; SP2/0-Ag14 (SP-2) [cf. Nature, 276, 269-270 (1978)]; P3-X63-Ag8653 (653) [cf. J. Immunol., 123, 1548-1550 (1979)]; and P3-X63-Ag8 (X63) [cf. Nature, 256 495-497 (1975)]. These cell lines are subcultured in an 8-azaguanine medium which is obtained by adding 1.5 mM of glutamine, 5 × $10^{-5}$ M of 2-mercaptoethanol, 10 $\mu$g/m$\ell$ of gentamycin and 10 v/v% of fetal calf serum to an RPMI-1640 medium to give a medium, which will be called the "normal medium" hereinafter, and further adding 15 $\mu$g/m$\ell$ of azaguanine thereto. These cell lines are subcultured into the normal medium three or four days before the cell fusion to thereby secure a cell count of at least 2 × $10^7$ on the day of the cell fusion.

(3) Cell fusion

The antibody-producing cells as immunized in (1) and the myeloma cells as obtained in (2) are thoroughly washed with the MEM medium or PBS. Then, these cells are mixed together in such a manner as to give a cell count ratio of the former to the latter of 5 to 10:1. The resulting mixture is centrifuged at 1,200 rpm for five minutes and then the supernatant is discarded. The precipitated cells are thoroughly loosened and 0.2 to 1 mℓ/$10^3$ antibody-producing cells of a mixture comprising 2 g of polyethylene glycol 1,000 (PEG-1,000), 2 mℓ of MEM and 0.7 mℓ of dimethyl sulfoxide are added thereto at 37°C under stirring. Then, 1 to 2 mℓ portions of MEM are further added thereto several times at one to two minute intervals to thereby adjust the total volume of the mixture to 50 mℓ. After centrifuging the mixture at 900 rpm for five minutes, the supernatant is discarded and the cells are slowly loosened. 100 mℓ of the normal medium RPMI-1640 containing 10 v/v% of FCS is added thereto and the cells are slowly suspended in the medium by inhaling and blowing through a volumetric pipet.

1 mℓ of the resulting suspension is pipetted into each well of a 24-well incubation plate and incubated in an incubator under 5% of $CO_2$ at 37°C for 24 hours. Then, 1 mℓ of an HAT medium, which is obtained by adding $10^{-4}$ M of hypoxanthine, $1.5 \times 10^{-5}$ M of thymidine and $4 \times 10^{-7}$ M of aminopterin to the normal medium, is added to each well and the plate is then incubated for an additional 24 hours. 1 ml of the incubation supernatant in each well is discarded and the same amount of fresh HAT medium is added at 24 hour intervals for the following two days. The incubation is continued in a $CO_2$ incubator at 37°C for 10 to 14 days.

In a well where fused cells form colonies, 1 ml of the supernatant is discarded and the same amount of an HT medium obtained by removing aminopterin from the HAT medium is added. The conversion into the HT medium is carried out at 24 hour intervals for the following two days.

After incubating the cells in the HT medium for three to four days, some portion of the incubation supernatant is collected and the antibody titer thereof against human pulmonary adenocarcinoma is determined according to the above-mentioned enzyme immunoassay. Then, the reactivity of the incubated cells with human normal cells, tissue and membrane components thereof are determined in the same manner. Thus, wells specifically reacting with human pulmonary adenocarcinoma cells or tissue or membrane components thereof are selected. Wells which do not react with human normal cells, tissue or membrane components thereof but which intensely react with human pulmonary adenocarcinoma cells or tissue or membrane components thereof are subjected to cloning twice by limiting dilution analysis to thereby select a cell line which shows a stable antibody titer against human pulmonary adenocarcinoma cells or tissue or membrane components thereof as an antihuman pulmonary adenocarcinoma monoclonal antibody-producing hybridoma line.

(4) Preparation of monoclonal antibody

2 to $4 \times 10^6$ antihuman pulmonary adenocarcinoma monoclonal antibody-producing hybridoma cells as obtained in (3) are intraperitoneally injected into a female nude mouse aged eight to ten weeks which has been treated with pristane by intraperitoneally administering 0.5 ml of 2,6,10,14-tetramethylpentadecane (pristane) followed by feeding for two weeks. After 10 to 21 days, the hybridoma cells become ascites cancer cells. The ascites fluid is collected from the mouse and centrifuged at 3,000 rpm for five minutes. After removing solid matter, the residue is salted-out with 50% saturated ammonium sulfate, dialyzed against a 0.04 M phosphate buffer solution (pH 8.0, containing 0.03 M of NaCl) and passed through a DE52 column (manufactured by Whatman). IgG fractions are collected to thereby give the desired monoclonal antibody in a purified state.

The isotype of the antibody is determined by Ouchterlony's method (double immunodiffusion) (cf. Menekigaku Numon, Seibutsu Kagaku Jikkenho 15, Gakkai Shuppan Center, p. 74 (1981)).

Protein is determined by Folin's reaction and the absorbance at 280 nm [1.4 ($OD_{280}$) ≒ 1 mg/mℓ of immunoglobulin].

The specificity of the monoclonal antibody thus obtained is determined by, for example, evaluating the reactivities with normal or tumor tissues or membrane components thereof originating from various human organs obtained from two or more samples; those with various human normal or tumor cell strains or fetal human cell strains or membrane components thereof; or those with known carcinoembryonic antigens such as CEA by enzyme immunoassay or immunohistological (PAP) methods. In any case, monoclonal antibody which scarcely reacts with antigens other than human pulmonary adenocarcinoma should be selected.

(5) Analysis of antigen

Upon the above-mentioned enzyme immunoassay or immunohistological staining, an antigen selected from pulmonary adenocarcinoma membrane components, pulmonary adenocarcinoma cell strains and pulmonary adenocarcinoma tissues is pretreated with an enzyme such as neuraminidase or protease or a reagent such as periodic acid and then reacted with the monoclonal antibody. Thus, the chemical properties of the antigen, i.e., the chemical properties of the antigen site recognized by the monoclonal antibody, are determined from the difference between the reactivity of the treated antigen, as described above, and that of the untreated antigen each with the monoclonal antibody. That is to say, it is assumed that sialic acid, protein or a sugar chain might relate to the antigen determinant when the antigenicity is lost by treating with neuraminidase, protease or periodic acid, respectively.

To further illustrate the present invention, the following Examples will be given.

## EXAMPLE 1

### (1) Preparation of antibody-producing cells

Normal human lung membrane components (100 $\mu$g/animal on a protein basis) were intravenously administered to newborn BALB/c mice (obtained from Shizuoka Jikken Dobutsu) within 24 hours after birth. Eight weeks later, 100 $\mu$g on a protein basis of a human pulmonary adenocarcinoma membrane fragment was intraperitoneally administered to each mouse together with 2 mg of aluminum hydroxide gel and $1 \times 10^9$ of dead B. pertussis vaccine cells. Subsequently the animal was immunized with 100 $\mu$g portions on a protein basis of the same antigen three to five times at one to two week intervals. Among these mice, those showing antisera highly reactive with human pulmonary adenocarcinoma cells or tissue or membrane components thereof were referred to immunized mice and spleen cells obtained therefrom were subjected to cell fusion.

### (2) Preparation of mouse myeloma cells

An 8-azaguanine resistant mouse myeloma cell line P3-U1 was incubated in the normal medium to give $2 \times 10^7$ or more cells which were used as parent cells at the cell fusion.

### (3) Preparation of hybridomas

The spleen cells and myeloma cells obtained in (1) and (2), respectively, were fused in the above-mentioned manner in a ratio of 5:1 and incubated in HAT medium at 37°C for 14 days under 5% of $CO_2$. Then, fused cells were selected and further incubated in an HT medium. The antibody titer against antihuman pulmonary adenocarcinoma of each well was determined and active wells were transplanted into a normal medium. After repeating cloning twice, a hybridoma cell line ALC-390 producing a monoclonal antibody, which would never react with normal human cells or tissues but would react with human pulmonary adenocarcinoma, was selected through an enzyme immunoassay method, an immunohistochemical staining method and an immunocytochemical staining method.

### (4) Purification of monoclonal antibody

$4 \times 10^6$ of the hybridoma cells ALC-390 as obtained (3) were intraperitoneally injected to female nude mice aged eight weeks which had been treated with pristane. 10 to 21 days thereafter, the hybridomas became ascites cancer cells. 5 to 10 m$\ell$ portions of the ascites fluids were collected from the animals showing standing ascites fluids. These acsites fluids were combined and centrifuged at 3,000 rpm for five minutes. After removing solid matter, the residue was salted out with 40% saturated ammonium sulfate and dialyzed against a 0.04 M phosphate buffer solution (pH 8.0, containing 0.03 M of NaCl). Then, it was passed through a DE52 column (manufactured by Whatman, bed volume: 50 m$\ell$) at a flow rate of 20 to 30 m$\ell$/hr. IgG fractions were collected to obtain the purified monoclonal antibody.

### (5) Specificity of ALC-390

Table 1 shows the reaction specificities of the antihuman pulmonary adenocarcinoma monoclonal antibody ALC-390 thus obtained.

The determination was carried out by ELISA in the following manner.

Utilization of tissue (membrane component) as target:

50 $\mu\ell$ of a 0.1 mg/m$\ell$ solution of the target tissue (membrane component) were pipetted into each well of a 96-well ELISA plate (manufactured by Linbro). The plate was allowed to stand at 37°C for two hours or at 4°C overnight to thereby fix the tissue (membrane component). After washing the plate with PBS, 100 $\mu\ell$ of PBS containing 10 v/v% of FCS was pipetted into each well to thereby protect the active residues of the fixed tissue (membrane component). After washing with PBS, 50 $\mu\ell$ of the primary antibody (ALC-390) was added to each well and the plate was allowed to stand at 37°C for one to two hours or at 4°C overnight, thus reacting the target with the antibody. Then, the plate was washed with PBS containing 0.05 v/v% of Tween-20 (manufactured by Wako Pure Chemical Industries Co.) five times to remove the unreacted antibody. 50 $\mu\ell$ of the secondary antibody, which was rabbit antimouse immunoglobulin binding to peroxdiase (manufactured by Miles-Yeda, diluted 200-fold), was added to each well and allowed to react at 37°C for one hour. The plate was washed with PBS containing 0.05 v/v% of Tween-20 five times and then with deionized water three times.

50 $\mu\ell$ of ABTS was added to each well to thereby initiate the reaction and 50 $\mu\ell$ of a 5 w/v% aqueous solution of sodium lauryl sulfate was added to terminate the reaction.

Utilization of cultured cells as target:

The target cells were incubated in a 96-well incubation plate (manufactured by Linbro). When the cells became confluent, the same reactions as those described above with respect to the case of target tissue (membrane component) were carried out except both of the primary and secondary antibodies were reacted at room temperature for 30 minutes. The reaction mixture was transported into a 96-well analysis plate after coloration to terminate the reaction.

When an antigen CEA was employed as the target, the above procedure was followed except that the tissue (membrane component) was replaced with CEA. In each case, the absorbance of the reaction mixture was determined at 415 nm while using that at 490 nm as a control.

## Table 1

| Antigen | Reactivity (ELISA) positive sample/ total sample |
|---|---|
| Tissue membrane component | |
| pulmonary adenocarcinoma | 7/10[a] |
| lung squamous cell carcinoma | 1/12 |
| normal lung | 0/6 |
| other normal tissues[b] | 0/11 |
| Cultured cell line | |
| lung cancer[c] | 3/8 |
| pancreatic cancer | 1/1 |
| colon cancer | 2/2 |
| stomach cancer | 3/3 |
| other cancers[d] | 4/5 |
| Antigen | |
| CEA | 0/1 |

a): Number of positive samples/Total number of samples.

b): Liver, kidney, stomach, pancreas, intestine, spleen, heart, medulla, lymphocyte, erythrocyte and brain.

c): Pulmonary adenocarcinoma: 2/3

d): Uterine cancer, prostate cancer and myeloma.

As shown in Table 1, ALC-390 was highly specific to pulmonary adenocarcinoma cells but did not react with CEA. These facts suggest that it is different from antiCEA antibody.

(6) Classification of the isotype of ALC-390

The isotype of antihuman pulmonary adenocarcinoma monoclonal antibody ALC-390 was determined by Ouchterlony's method. As a reuslt, it is found that the antibody ALC-390 belongs to IgG class, more specifically, IgG$_1$ class.

EXAMPLE 2

Sections of various normal adult and fetal organs and cancer tissues, which were fixed with formalin, embedded in paraffin and sliced with a microtome at a thickness of 5 $\mu$m, were each fixed on a glass slide coated with egg white albumin. After removing the paraffin with xylene, each section was made hydrophilic stepwise using alcohol water. Then, each section was rinsed with deionized water for five minutes and allowed to stand in methanol containing 0.3% of $H_2O_2$ at room temperature for 30 minutes to block endogenous peroxidase. Then, the section was washed with PBS for 20 minutes and allowed to stand in diluted normal equine serum at room temperature for 20 minutes. After removing the excessive serum, the section was reacted with the primary antibody (antihuman pulmonary adenocarcinoma monoclonal antibody ALC-390, 10 $\mu$g/m$\ell$) for 30 minutes. After washing, the reaction mixture was reacted with a diluted biotinized antibody biotinized rabbit antIgG antibody) for 30 minutes. After washing, it was further reacted with an avidin/biotin/peroxidase complex (manufactured by Vector) for 30 minutes. Then, the reaction mixture was thoroughly washed and reacted with a peroxidase substrate, which was a 0.1% solution of diaminobenzidine tetrahydrochloride in a 0.1 M tris hydrochloride butter solution (pH 7.2) containing 0.02% of $H_2O_2$, for two minutes; the reaction was stopped by ice-cooling. After staining with hematoxylin and dehydrating with an aqueous alcohol and xylene solution, the residue was fixed with Canada balsam and observed under a microscope.

The results are shown in Table 2.

## Table 2

| Tissue | Positive samples/ total samples | Positive ratio (%) |
|---|---|---|
| pulmonary adenocarcinoma | 7/9* | 77.8 |
| lung squamous cell carcinoma | 2/11** | 18.2 |
| lung large cell carcinoma | 3/6** | 50.0 |
| lung small cell carcinoma | 0/7 | 0.0 |

*: Cell membrane is highly reactive.

**: Some cancer cells are slightly reactive.

As shown in Table 2, ALC-390 slightly reacted with lung squamous cell carcinoma and some cells of a lung large cell carcinoma tissue but reacted intensely with pulmonary adenocarcinoma at a high ratio. It was very slightly reactive with some cells of normal or fetal tissues including heart, pancreas, stomach, large intestine, kidney, thyroid body, brain, spleen, liver and small intestine, but showed no reaction with other tissues.

EXAMPLE 3

The antigen determinant recognized by the monoclonal antibody ALC-390 was analyzed by reacting ALC-390 with pulmonary adenocarcinoma membrane components which was each treated with the following enzymes and reagents prior to the reaction.

Enzymes and Reagents:

Trypsin (GIBCO, 2.5 w/v% solution):
dissolved in PBS to give a final concentration of 0.25 w/v%.
Neuraminidase (Boehringer Mannheim):
dissolved in 0.1 M acetate buffer (pH 4.5) containing 3 mM $CaCl_2$ to give a final concentration of 0.1 U/m$\ell$.
$\alpha$-L-Fucosidase (Boehringer Mannheim):
dissolved in 0.1 M phosphate buffer (pH 6.3) to give a final concentration of 0.1 U/m$\ell$.

Protease (Sigma):
dissolved in 0.1 M phosphate buffer (pH 7.2) to give a final concentration of 10 U/mℓ.
NaIO₄ (Wako Pure Chemical):
dissolved in PBS to give a final concentration of 50 mM.

Pulmonary adenocarcinoma membrane components (100 $\mu$g protein/ml) were dispensed in 50 $\mu$l portions into each well of an EIA plate (Linbro). After allowing the plate to stand overnight at 4°C, each well was washed with PBS three times. Then, 200 $\mu\ell$ of 1 w/v% BSA-PBS was pipetted into each well and the plate was allowed to stand at room temperature for 0.5 to 2 hours. After washing each well with PBS three times, the above-described enzyme solution or reagent solution was each dispensed in 50 $\mu\ell$ portions into each well and allowed to react at 37°C for an hour. Then, each well was washed with PBS five times and 50 $\mu\ell$ of the monoclonal antibody ALC-390 (10 $\mu$g/mℓ) was pipetted into each well, followed by allowing the plate to stand overnight at 4°C.

Then, each well was washed with Tween 20-PBS (Tween 20: Wako Pure Chemical) five times and 50 $\mu\ell$ of peroxidase-labeled mouse IgG (400-fold dilution) was pipetted into each well. After reaction at room temperature for 2 hours, each well was washed with Tween 20-PBS five times. ABTS substrate solution was dispensed in 100 $\mu$l portions into each well and allowed to react at room temperature for 30 minutes, and thereafter the absorbance of the reaction mixture in each well was measured at 415 nm.

As shown in Fig. 1, the antigenicity was lost by treatment with trypsin and protease. Therefore, it is assumed that the monoclonal antibody ALC-390 recognizes protein as the antigen determinant.

While the invention has been described in detail and with reference to specific embodiments thereof, it will be apparent to one skilled in the art that various changes and modifications can be made therein without departing from the spirit and scope thereof.

## Claims

1. An antihuman pulmonary adenocarcinoma monoclonal antibody ALC-390 (ECACC 87032601) which belongs to IgG class and is reactive with human pulmonary adenocarcinoma cell membranes and recognizes a protein epitope as the antigenic determinant.

2. A method of detecting the presence of human pulmonary adenocarcinoma in a patient sample comprising:

    (a) subjecting a serum sample from the patient to an enzyme immunoassay with the monoclonal antibody ALC-390 (ECACC 87032601) that (1) belongs to the class IgG, (2) recognizes a protein epitope as the antigenic determinant and (3) is reactive with human pulmonary adeoncarcinoma cell membranes and (4) is devoid of reactivity with normal human lung tissue; and thereafter
    (b) examining the results for the presence of a positive reaction.

3. A diagnostic method of distinguishing human pulmonary adonocarcinoma cells from other cells including lung squamous cell carcinoma cells, lung large cell carcinoma cells, lung small cell carcinoma cells and normal human lung cells, said diagnostic method comprising the steps of:

    (a) subjecting a patient sample to an enzyme immunoassay with the monoclonal antibody ALC-390 (ECACC 87032601) that (1) belongs to the class IgG, (2) selectively recognizes a protein epitope as the antigenic determinant and (3) is substantially more reactive with human pulmonary adenocarcinoma cell membranes than lung squamous cell carcinoma cells, lung large cell carcinoma cells, lung small cell carcinoma cells and thereafter
    (b) assessing the degree of reactivity, if any, of the monoclonal antibody with the cells of the patient sample.

4. Hybridoma cell line ALC-390 (ECACC 87032601).

5. A pharmaceutical composition comprising an effective amount of an antibody as defined in claim 1, optionally in combination with a pharmaceutically acceptable carrier or adjuvant.

6. The use of an antibody as defined in claim 1 for preparing a pharmaceutical composition for the treatment of pulmonary adenocarcinoma.

EP 0 285 143 B1

**Patentansprüche**

1. Gegen menschliches Lungenadenocarcinom gerichteter monoklonaler Antikörper ALC-390 (ECACC 87032601) der Klasse IgG, der mit Zellmembranen des menschlichen Lungenadenocarcinoms reagiert und ein Proteinepitop als antigene Determinante erkennt.

2. Verfahren zum Nachweis der Präsenz von menschlichem Lungenadenocarcinom in einer Probe aus einem Patienten, wobei man:
   (a) eine Serumprobe des Patienten einem Enzymimmunoassay mit dem monoklonalen Antikörper ALC-390 (ECACC 87032601) unterzieht, der (1) zur Klasse IgG zählt, (2) ein Proteinepitop als antigene Determinante erkennt, (3) mit Zellmembranen von menschlichem Lungenadenocarcinom reagiert und (4) keine Reaktivität gegenüber normalem menschlichem Lungengewebe zeigt; und anschließend
   (b) die Ergebnisse auf Vorliegen einer positiven Reaktion überprüft.

3. Diagnostisches Verfahren zur Unterscheidung von menschlichen Lungenadenocarcinomzellen von anderen Zellen, umfassend Squamazellen-Lungencarcinomzellen, großzellige Lungenadenocarcinomzellen, kleinzellige Lungenadenocarcinomzellen und normale menschliche Lungenzellen, wobei man bei diesem diagnostischen Verfahren:
   (a) eine Probe eines Patienten einem Enzymimmunoassay mit dem monoclonalen Antikörper ALC-390 (ECACC 87032601) unterzieht, der (1) zur Klasse IgG zählt, (2) ein Proteinepitop als antigene Determinante selektiv erkennt und (3) mit menschlichen Lungenadenocarcinom-Zellmembranen im wesentlichen stärker reagiert als mit Squamazellen-Lungencarcinomzellen, großzelligen Lungencarcinomzellen, kleinzelligen Lungencarcinomzellen, und anschließend
   (b) den gegebenenfalls vorliegenden Reaktivitätsgrad des monoklonalen Antikörpers mit den Zellen der Patientenprobe bestimmt.

4. Hybridomzellinie ALC-390 (ECACC 87032601).

5. Pharmazeutisches Mittel, umfassend eine wirksame Menge eines Antikörpers gemäß Anspruch 1, gegebenenfalls in Kombination mit einem pharmazeutisch verträglichen Träger oder Adjuvans.

6. Verwendung eines Antikörpers gemäß Anspruch 1 zur Herstellung eines pharmazeutischen Mittels zur Behandlung von Lungenadenocarcinom.

**Revendications**

1. Anticorps monoclonal anti-adénocarcinome bronchique humain ALC-390 (ECACC 87032601) qui fait partie de la classe des IgG, réagit avec les membranes des cellules d'adénocarcinome bronchique humain et reconnaît un épitope protéique comme étant le déterminant antigénique.

2. Procédé de détection de la présence d'un adénocarcinome bronchique humain dans un échantillon d'un malade, comprenant les étapes consistant à :
   (a) soumettre un échantillon sérique du malade à un dosage immunoenzymatique à l'aide de l'anticorps monoclonal ALC-390 (ECACC 87032601) qui (1) fait partie de la classe des IgG, (2) reconnaît un épitope protéique comme étant le déterminant antigénique, (3) réagit avec les membranes des cellules d'adénocarcinome bronchique humain et (4) est dépourvu de réactivité vis-à-vis du tissu bronchique humain normal ; et ensuite
   (b) examiner les résultats pour déceler la présence d'une réaction positive.

3. Procédé diagnostique permettant de distinguer des cellules d'adénocarcinome bronchique humain d'autres cellules, dont des cellules de cancer épidermoïque bronchique, des cellules de cancer bronchique à grandes cellules, des cellules de cancer bronchique à petites cellules et des cellules de poumon humain normal, ledit procédé diagnostic comprenant les étapes consistant à :
   (a) soumettre un échantillon d'un malade à un dosage immunoenzymatique à l'aide de l'anticorps monoclonal ALC-390 (ECACC 87032601) qui (1) fait partie de la classe des IgG, (2) reconnaît sélectivement un épitope protéique comme étant le déterminant antigénique et (3) est sensiblement plus réactif avec les membranes des cellules d'adénocarcinome bronchique humain qu'avec les

11

cellules de cancer épidermoïque bronchique, les cellules de cancer bronchique à grandes cellules, les cellules de cancer bronchique à petites cellules, et ensuite

(b) déterminer le degré de réactivité, s'il existe, de l'anticorps monoclonal vis-à-vis des cellules de l'échantillon du malade.

4.  Lignée cellulaire d'hybridome ALC-390 (ECACC 87032601).

5.  Composition pharmaceutique comprenant une quantité efficace d'un anticorps tel que défini dans la revendication 1, facultativement associé à un véhicule ou adjuvant pharmaceutiquement acceptable.

6.  Utilisation d'un anticorps tel que défini à la revendication 1, pour la préparation d'une composition pharmaceutique destinée au traitement de l'adénocarcinome bronchique.

Fig. 1